(19) Europäisches Patentamt
European Patent Office
Office européen des brevets

(11) **EP 3 480 821 B1**

(12) **EUROPEAN PATENT SPECIFICATION**

(45) Date of publication and mention
of the grant of the patent:
**27.04.2022 Bulletin 2022/17**

(21) Application number: **18184310.3**

(22) Date of filing: **18.07.2018**

(51) International Patent Classification (IPC):
**G16H 10/20** (2018.01)     **G16H 10/60** (2018.01)

(52) Cooperative Patent Classification (CPC):
**G16H 10/20; G16H 10/60**

(54) **CLINICAL TRIAL SUPPORT NETWORK DATA SECURITY**

DATENSICHERHEIT IM NETZWERK FÜR KLINISCHE STUDIEN

SÉCURITÉ DES DONNÉES D'UN RÉSEAU DE SUPPORT D'ESSAI CLINIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.11.2017 EP 17199576**

(43) Date of publication of application:
**08.05.2019 Bulletin 2019/19**

(73) Proprietor: **Icon Clinical Research Limited
Dublin 18 (IE)**

(72) Inventors:
• **FOX, Ronan**
  **Dublin 18, (IE)**
• **KELLY, Sean**
  **Dublin 18, (IE)**
• **O'LEARY, Thomas**
  **Dublin 18, (IE)**

(74) Representative: **Weldon O'Brien Ltd.
Shannon Lodge
Casement Road
Bandon
County Cork, P72 TN24 (IE)**

(56) References cited:
**US-A1- 2001 027 331     US-A1- 2015 161 397**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Prior Art Discussion

The invention relates to management of clinical trials, and particularly to computer processing of data concerning patients taking part in trials managed by a clinical trial support network.

Clinical trials require gathering of considerable volumes of data from a wide variety of patients, both relating to medical events specific to medication being trialled and of a personal nature concerning the patients. It is of course very important that sensitive data specific to patients be managed very carefully to avoid breaches of security.

US2005/0065824 (Kohan) describes an approach in which a computer application audits data fields to determine those for encoding, and determines parameters for encoding. This approach provides structured data fields being anonymised for transfer between vendors in a healthcare system.

US2004/0199781 (Erickson) describes an approach in which comparisons are performed of data from sources and extraction is used to provide k-anonymity values. This uses k-anonymisation or bucketing of data from large sets of records so that no one record can be tracked back to an individual entity.

US8715180 (Medtronic) describes an approach to data privacy in which data is parsed and stored in first or second storage areas, and there is controlled access to the storage areas.

US8606746 (Oracle) describes a policy hub for maintaining and managing customer privacy information and privacy preferences and a rules database provides privacy rules. The policy hub spans the enterprise and allows creation of rules and privacy preferences based on such things as opt in/opt out for a customer for emails etc.

US8650645 (McKesson Financial Holdings) describes an approach in which hashing of files is performed to protect proprietary data. This is to protect data as part of an approval process where the hashes generated allow further processing of the data for approval.

US2006/0059149 (Dunki) describes use of a productive database in which records are anonymized, the records including non-static data elements generated or processed by programs and static elements which are invariable. The intent behind this approach appears to be to provide anonymised data so that is can simulate real world data during the development and testing of a computer application. The anonymised data is achieved by "masking" the productive data with data sourced from a second database of "noisy data". The result of this is a snapshot of data that is then used statically within a development/test environment.

US2001/0027331 (Thompson) describes a variable encryption scheme for data transfer between medical devices and related data management systems.

US2015/0161397 (Microsoft) describes management of sensitive production data.

The invention is directed towards providing data privacy or security with less impact on data processing in the clinical trial support network. Another object is to achieve more comprehensive data security.

Additional References

1. Term-weighting approaches in automatic text retrieval. Salton, Gerard, and Christopher Buckley. 24, 1988, Vol. Information processing & management. 5.
2. An information-theoretic perspective of tf-idf measures. Aizawa, Akiko. 39, 2003, Vol. Information Processing & Management. 1.
3. International Telegraph and Telephone Consultative Committee (CCITT). Information Technology - Open Systems Interconnection - Systems Management: Alarm Reporting Function. International Telecommunication Union. [Online] 10 Feb 1992. [Cited: 27 June 2017.] https://www.itu.int/rec/T-REC-X.733/en.

SUMMARY OF THE INVENTION

We describe a method performed by a clinical trial network comprising digital data processors, user interfaces, and databases, the method being as set out in the appended claim 1.

Other aspects of the method are set out in the appended claims 2 to 9.

The invention also provides a clinical trial network as set out in the appended claim 10.

## DETAILED DESCRIPTION OF THE INVENTION

### Brief Description of the Drawings

The invention will be more clearly understood from the following description of some embodiments thereof, given by way of example only with reference to the accompanying drawings, in which:

Fig. 1 is a diagram showing a clinical trial network of computers, servers and databases of the invention;

Fig. 2 is a flow diagram illustrating a workflow for setting up clinical trial data structures so that there is anonymization of data of patients both contributing to clinical trials and in receipt of communication from the network; and

Fig. 3 is a flow diagram showing real time steps in operation of the network to ensure data privacy.

### Description of the Embodiments

### Network

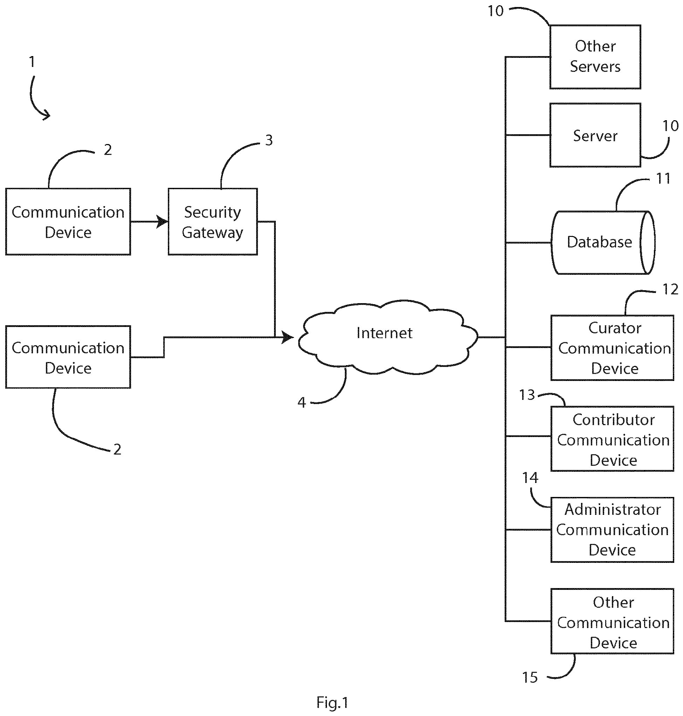

Referring to Fig. 1 a clinical trial support network 1 comprises communication devices 2 linked by a security gateway 3 to the Internet 4. This in turn links the devices 2 with servers 10, a database 11, a curator communication device 12, a contributor communication device 13, an administrator communication device 14, and with other communication devices 15. Each device and server comprises digital data processors and communication interfaces as is well known in the art. The database software comprises a relational database management system which supports a schema required to hold data relating to patient data postings including, but not limited to:

- The posting content.
- Identity of the person who posted the message
- Time and date of the posting
- Destination of the posting, such as the patient's clinical trial group, specific member of the group, or other "parent" content (such as the parent blog post if this was a comment on that original "parent" posting")
- Any extracted metadata about the posting, including automatically extracted keywords, manually added tags, flags to indicate the output of initial automated privacy analysis, and structured data resulting from natural language processing and automated semantic keyword extraction.

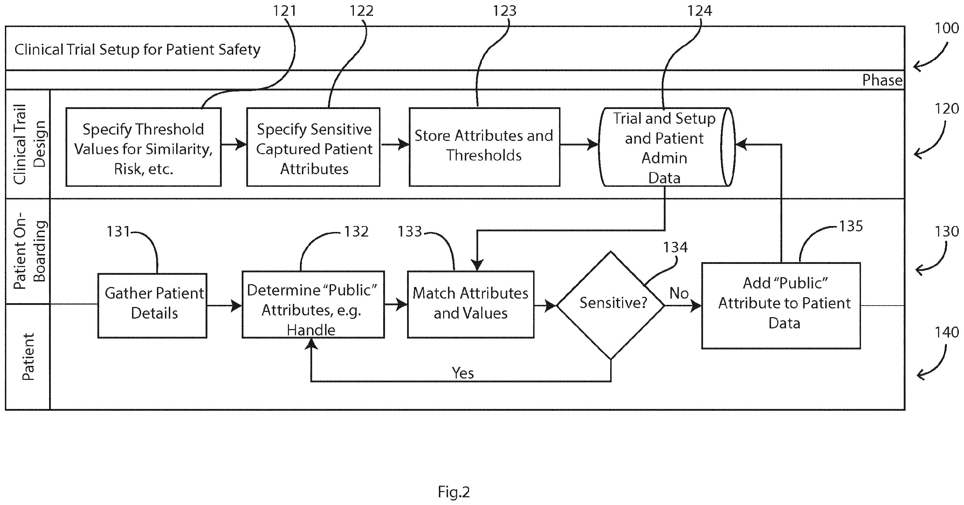

The servers are configured in hardware terms according to the specified requirements in the clinical trial network. In one example the servers have a speed in the range of 2 to 3 GHz, have in the range of 4 to 16 cores, and a memory capacity of 12 to 15 GB. However, the parameters may be different, depending on the capacity requirements.
The support network data processors are programmed to perform patient data attribute classification during clinical trial setup (120 in Fig. 2) and patient setup (130, 140 in Fig. 2) by identifying attributes which are sensitive, the attributes being instantiated as terms in clinical trial records. This may be done irregularly, such as once a month or when a re-configuration is desired. For each identified attribute a risk value or weighting associated with the attribute is stored.
The network devices and servers are programmed as follows for automatic processing of potential postings.
The database server 11 is configured to persist the posting and any associated data that may be generated through the administration of the clinical networks, inputs from any contributors, and outputs from any automatic or manual processing performed on the postings and related metadata. The database technology may be instantiated as a document store, and/or as an RDBMS, or as an in-memory data grid spanning clusters of servers to allow for faster throughput and real time processing of postings as they are made to the patient support network. The deployment of the database server may be in a private data centre on in a secured public cloud infrastructure to allow for quick scale up during periods of intense activity in a clinical trial and where the volumes of posts to be analysed may spike during these periods.
The curator communication device 12 provides a means for an "owner" or curator of the network site to perform tasks relating to the quality of the data on site, and also relating to privacy associated with any publicly-generated and available data. This includes providing a means to decide upon and enact processes relating to the publishing or deletion of data as required in the social network, including applying risk weightings to attributes as described in detail below. This device in turn may be part of a peer to peer network of devices which would allow for many people to collaborate in a workflow of authorisations and delegations to allow for multiple opinions and human oversight.
The contributor communication device 13 may be used by professional (often clinical) staff to provide content into the network 1. This content may be of a medical and/or regulatory nature.

The administrator communication device 14 enables a user to administer the network 1. Tasks such as account provisioning and subsequent management are performed by this device.

Other communication devices 15 enable other actors who may interact with the network 1 in roles not described above to for example, link sites, carry out research, etc.

Other servers 10 include servers that may be used in the provisioning of the network 1. Examples of these servers may be firewalls, load balancing servers, and redirection servers which may be required to provide scale, robustness and other non-functional services. As before these servers may be part of a cluster of (virtual) machines which can be deployed as required to satisfy the scale needs of the network.

Clinical Trial Design and Patient On-boarding

Referring to Fig. 2 a workflow 100 is implemented by the server 10 and the database system 11 of the network 1. There are stages as follows:

> 120, clinical trial design;
> 130, patient data on-boarding; and
> 140, patient data analysis and enrichment during configuration.

The workflow 100 concerns patient setup and includes data relating to a specific patient allowing not just attributes but also specific data values to be marked as sensitive, so that it can be used in the real time processing. There may be a temporal overlap as patients can be on-boarded as entries are being processed, but the aspects illustrated in Fig. 2 all relate to the early clinical trial or study setup and the on-boarding phase of each patient.

In the stage 120 a step 121 specifies threshold values for similarity and risk. Examples are shown in the table below with attributes, example values, and risk/weightings.

In step 122 sensitive patient attributes are specified. The threshold values of step 121 and the attributes are both stored in step 123 into a database 124 of trial set-up and patient administration data.

In more detail, during setup the trial designer decides upon a set of attributes which are considered as sensitive. An attribute is a type of term to be used in a patient record, such as Name or Address or Age, or any term which may be received in a potential posting. Each attribute is given a risk weighting which indicates the risk associated with protecting the privacy of the patient. An example of this attribute selection and weighting is as follows:

| Attribute | Example Value | Risk Weighting |
|---|---|---|
| **Patient Name** | John Doe | .5 |
| **Patient Age** | 37 | .03 |
| **Patient Street and Number** | 41 Appleton Way | .4 |
| **Patient County/City** | New York | .01 |
| **Patient Spouse Name** | Mary Doe | .05 |
| **Clinical Site Attending** | 42nd Street | .01 |

During patient on-boarding for a trial the attributes for patient details are gathered in step 131, and in step 132 public attributes are determined according to a decision step 134. In step 133 the attributes are matched to values in data retrieved from the database 124. This is fed into the decision step 134 for automatic updating of public attributes. Finally, in step 135 public attributes are added to the patient data of the database 124.

It will be appreciated from Fig. 2 that there is flexibility to allow manual specification of those attributes gathered about the patient in the current clinical trial which are deemed as being sensitive. This provides a taxonomy of search terms that will drive elements of the patient safety maximisation algorithms of the network. The weighting values are between 0 and 1.

Because all attributes are not equally risky for patient data privacy, the weighting between (0, 1) achieves excellent flexibility.

A set of "standard" terms is used to give a measure of the sentiment associated with specific postings.

As accounts are being set up by patients or on their behalf a "handle" is specified which will not contain any of the sensitive attributes identified in the setup phase. This handle is used by the patient when engaging in the online patient support network within that specific clinical trial.

The "handle" is chosen by the patient but must meet eligibility criteria to ensure that there is no connection to sensitive attributes.

The workflow of Fig. 2 can be enacted as part of the trial setup by study staff or it could form part of a self-service model where the patient executes some of the setup on his/her own behalf.

In the decision step "Sensitive?" 134, a form of NLP using an Apache Lucene index is executed. The specific Lucene search is configurable by use of a FuzzyQuery and can use weightings and other parameters to adjust the type of search done in the query using edit distances, as one example.

As part of the "Sensitive?" 134 step, this type of query will remove any commonly used words, combinations of words and word terms which might be specified by the patient or study team during public persona setup in the clinical trial setup phase (or patient recruitment phase). Also, the "Sensitive?" step 134 can also be used to ensure that anonymity of the participants (patients) is maintained with no sensitive data relating to patient identity being published.

Given that the identity of the patient who is posting a contribution to the support network is not known from the decision made when choosing the handle, the remaining task is to ensure that the specific content itself does not expose significant personal information to others on the trial.

Real Time Analysis

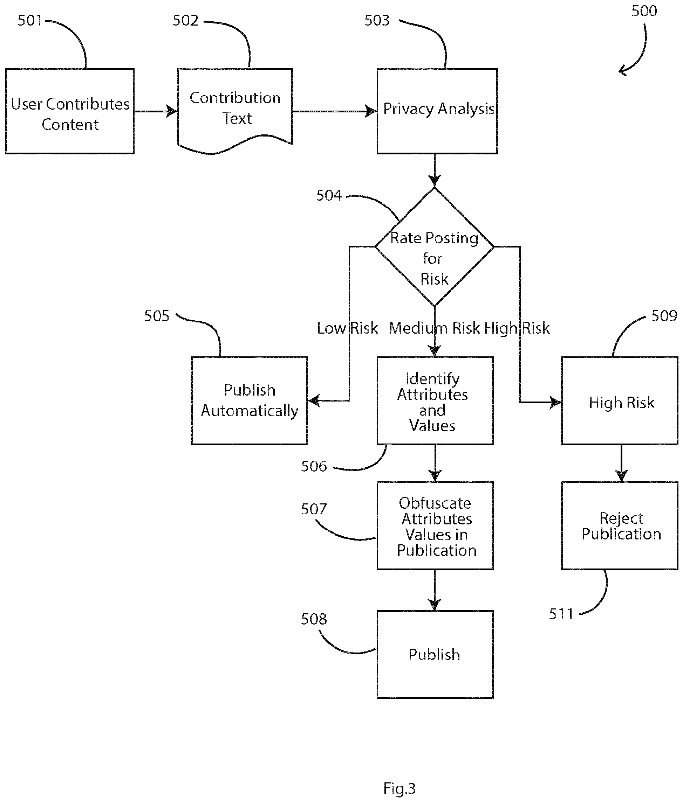

Fig. 3 shows a real-time process 500 that is performed automatically upon calculation of the risk associated with a submitted contribution or "potential posting" from a patient. Specific steps can be customized by use of configurable workflow tools. Advantageously, the network 1 has a customizable filtering mechanism to rate the risk associated with a proposed posting within a clinical trial network, despite the fact that the potential posting includes unstructured data (free form text).

After this initialization, in real time (501 and 502 in Fig. 3) a potential data posting is received, such as from a patient using an online portal or from a doctor managing a clinical trial group. Any of the servers 10 to 15 or any device 2 may provide such a contribution.

The data processors perform real time privacy analysis in step 503 for the potential posting, by using the risk values of attributes for which there are terms in the potential posting, to determine an overall risk level for the potential data posting. This analysis of a posting is done when the server receives the posting content, upon which the workflow in Fig. 3 begins. The processors may be spun up as required and determined by the scale needs of the network and the volume of postings being submitted. This would be enabled through a micro services architecture which will utilise self-healing and annealing infrastructures, deploying new instances as detected by management software (where service drop offline or where the incoming traffic is too much for the existing number of services deployed).

The data in the posting is unstructured, even though it may be provided via a guided patient interface form. There may be fields in a posting form to allow the patient to enter other data such as tags, or a "protect me" flag which might indicate to the curator that the patient is not sure whether they should do this posting, and that the curator should look at it prior to publication even if it passes through the automatic analysis. The data processor recognises such tags and filters them out because they don't need to be processed as part of step 503. The fact that the patient activates a tag indicating a patient-perceived risk is recorded as part of an audit process. In some embodiments activation of such a tag by the patient causes the privacy analysis step 503 to be performed differently by marking the posting as Medium Risk and moving straight to the "Rate Posting for Risk" step (504) where the curator will have the opportunity to review the content as obfuscated by the system prior to publication.

The database server digital processors execute an algorithm for measuring the risk associated with these terms, as follows:

$$risk(t,d) \approx \max_{0 \leq x \leq 1} \sum tfidf(t,d,D) = \max_{0 \leq x \leq 1} \sum_{t}^{n} tf(t,d) * idf(t,D)$$

The term frequency part of the algorithm is modified to take into account the privacy risk or importance associated with the specific attribute:

$$tf(t,d) = \sum_{i=1}^{n} tf_i * weight_i \Big/ T$$

where *tf(t,d)* is a modified measure of the term frequency associated with a posting in a document d,

$tf_i$ is a count of instances of a term $i$ in the posting,

weight $_i$ is the risk or importance of the term $I$, and

$T$ is the total count off instances of the terms identified as being potentially sensitive occurring in the current document.

As noted above a "term" is an instance of an "attribute".

Inverse document frequency is a measure of how unique the term is across all other postings published to date, and thus how much information is exposed by use of the attribute in a specific posting. It is given as:

$$idf(t, D) = \log \frac{N}{1 + |\{d \in D, t \in d\}|}$$

where $N$ is the total number of published postings

and $|\{d \in D, t \in d\}|$ is the number of postings where the term $d$ appears

Then, the maximum operator is applied to capture situations where the *idf* will identify a high weighting and push the *idf* and thus the risk above 1.

The data processors, when executing algorithms to analyse a potential posting, use additional criteria including, but not limited to:

term frequency in the posting; and/or

count of instances of each term in the posting; and/or

a total count of instances of terms which are potentially sensitive.

Other parameters include the risk or importance associated with the term such that if it was released, it would have a measurable risk of revealing sensitive patient data.

The process may be performed in an offline batch mode. In this example, it might for efficiency run when there are batch sizes of 20 or 30 messages, and release together.

The step 503 results in a step 504 of posting a risk value. Referring also to the ranges in the table below there is:

505, automatic publication if risk is low

506, 507, 508, for medium risk attribute identification, obfuscating values followed by publication (manual intervention may also feature here depending on the setup of the trial);

509, 511, determining high risk and rejecting publication.

| Risk Range | Meaning | Action |
|---|---|---|
| $0 \leq x \leq 0.4$ | Low Risk | Publish Automatically |
| $0.4 \leq x \leq 0.7$ | Medium Risk | Send for Review prior to Publishing |
| $0.7 \leq x \leq 1$ | High Risk | Do not Publish |

Table of Step 504 Posting Risk Ranges

The step of obfuscating values and terms is very advantageous because it means that there is an efficient flow of postings which are safe, avoiding excessive feedback to patient devices. The posting may be an unaltered version of the original posting, or an edited version of the posting to remove private data.

This step is performed as follows:

The identified instance of the sensitive data (for example, the patient name) is either replaced with a random set of characters to prevent any possible linkage to the patient name

The identified instance could also be replaced with a pre-configured string which may have been specified as part of the study/patient setup. At that point the curator may be given the opportunity to specify fixed replacement values for each sensitive attribute found.

It will be appreciated that, because a risk is determined during setup for each potentially sensitive attribute, real time dynamic decision making is performed as set out in Fig. 3 to ensure privacy. There is therefore very little impact on performance of the clinical trial network.

Once the analysis is performed the potential posting is transmitted or posted according to the result: blocked, full publication, or partial/obfuscated publication.

This network could also identify adverse side effects that a trial is having on patients by identifying conditions using the above taxonomy and calculating a distribution across the patient set. For example, a patient may declare that he/she is experiencing a side effect of a rash while accepting the therapy associated with the trial. A server can use Natural Language Processing (NLP) techniques to identify this side effect through multiple postings from multiple patients either automatically, or through the intervention of a human curator. These NLP techniques are well established in the literature and extend to semantic analysis of keywords based on either publicly available dictionaries and thesauri through to more limited analysis carried out in the form of taxonomy lookups through similarities, stemming, and other well-established methods. This would then initiate that globalised search. From this the trial may also discover the remedies patients are using to treat such rashes which may improve the patient experience and thus the likelihood of them staying on the study until completion. Once a condition is identified further symptoms can be added and applied to historical data to further and quickly ascertain the impact of such side effects supporting immediate action.

The network may identify and filter comments directed at other contributors which could be addressed by extending the taxonomy of keywords to identify such sensitive concepts as "placebo" and "rash". In a social networking site or a blog site where patients can comment on articles or other comments it would be important to discover and potential curate such entries as might indicate to a patient that they may be on one arm or another within a study. A patient could discover from comments or other postings that they might be taking a placebo and thus upon that realisation leave the trial before completion.

The network can also protect trial-sensitive information from making its way into the public domain, enforcing trial protocol on such matters.

The network could be used to enable inter-site as well as inter-patient social networks where the patent can protect sites from exposing patient or other sensitive data.

Advantages

The network incorporates structured data fields which are anonymised by allowing their identification and configuration and extending that usage to identifying the specific values within those fields to determine if there is disclosure of patient-sensitive data with unstructured information released by the patient into a social network environment linked with the clinical trial support network 1. It does not need to use bucketing and is focused on the risk associated with unstructured data (free form text) generated by the patient.

The network does not need to use a configuration or set of preferences per patient which are used to identify identifiable information. It is capable of very efficiently and effectively processing unstructured patient communications and with a set of patient identifiable data elements protect patient data in an online social network for example.

Also, the network provides a mechanism to protect unstructured data from revealing any sensitive information from a patient, primarily by executing the risk algorithm which forces the data through a review and approval process, but does not use hashing to do so as part of the workflow. Also, the technology of the invention relies on real time ingestion and anonymization of patient data without necessarily having a historical data set with which to work. Rather, it uses profiles and risk assessment to identify potential data elements that could be used to identify a patient at risk and to then redact that information from a real-time interaction such as through a clinical trial support network.

The algorithms executed by the digital data processors for automatically determining a risk value for a posting use the parameters of:

- a modified measure of term frequency in a posting,
- count of instances of the term in a posting;
- a weight reflecting risk or importance of the term;
- a total count of instances of an attribute in a posting.

The total count parameter may be omitted.

**Claims**

1. A method performed by a clinical trial network (1) comprising digital data processors (10-15) user interfaces, a database server, and databases (11), wherein the method (100) comprises steps performed by at least one said digital data processor including:

  (a) performing patient data attribute classification during clinical trial setup (120) by identifying (121, 122) attributes which are sensitive, said attributes being instantiated as unstructured terms in clinical trial records, and applying a handle for use by a patient when engaging with the clinical trial network, said handle not having any of said sensitive attributes;

(b) providing a sensitivity risk weighting (132, 133) associated with each of a plurality of attributes, the risk weighting representing a risk value for the associated attribute and being in a range from a minimum to a maximum;

(c) receiving (501, 502) a potential data posting;

(d) performing real time privacy analysis (503) of the potential data posting, by identifying terms and posting parameters and using said risk weightings of attributes to determine a posting data risk level (Low, Medium, High) for the potential data posting,

a parameter is one or more selected from:

extracted metadata about the potential posting,
a set of automatically-extracted keywords,
a tag added or selected manually by a patient,
a flag to indicate the output of a preliminary automated privacy analysis, and
structured data resulting from natural language processing and automated semantic keyword extraction,

wherein the parameters in step (d) include derived parameters for the potential data posting to determine said posting risk level, in which a derived parameter is one or more selected from:

an unstructured term frequency in the posting,
a count of instances of each unstructured term in the posting, and
a total count of instances of unstructured terms which are potentially sensitive,

wherein the real time privacy analysis (503) is performed by a server executing an algorithm as follows:

$$risk(t,d) \approx \max_{0 \leq x \leq 1} \sum tfidf(t,d,D) = \max_{0 \leq x \leq 1} \sum_{t}^{n} tf(t,d) * idf(t,D)$$

in which the term frequency part of the algorithm is modified to take into account the privacy risk or importance associated with the specific attribute:

$$tf(t,d) = \sum_{i=1}^{n} tf_i * weight_i / T$$

where *tf(t,d)* is a modified measure of the term frequency associated with a posting in a document d,
$tf_i$ is a count of instances of a term *i* in the posting,
weight $_i$ is the risk or importance of the term *I*, and
*T* is the total count off instances of the terms identified as being potentially sensitive occurring in the current document,
and in which *idf(t,D)* denotes inverse document frequency, which is a measure of how unique a term is across all other postings published to date, and thus how much information is exposed by use of the attribute in a specific posting, and is given as:

$$idf(t,D) = \log \frac{N}{1 + |\{d \in D, t \in d\}|}$$

where *N* is the total number of published postings
and |{d ∈ *D*, *t* ∈ d}| is the number of postings where the term *d* appears,

(e) posting data (505, 508, 509) according to the privacy analysis of step (d) as:

if said risk level is low, an unaltered version (505) of the original potential posting;

if the risk level is medium, an edited version (508) of the original potential posting;
if the risk level is high, reject the potential data posting; and

wherein the at least one digital data processor automatically executes steps (d) and (e) for each potential data posting, and
wherein the database server (11) persists the received potential data posting and associated data including inputs from contributors related to the received posting, and metadata, and also outputs for the received posting arising from step (d).

2. A method as claimed in claim 1, wherein one or both of the steps (a) and (b) are performed in response to processor-guided input of data by a patient according to a patient data interface, and wherein step (a) is performed per clinical trial as part of a clinical trial design phase (120).

3. A method as claimed in claims 1 or 2, wherein said at least one digital data processor is configured to analyse a plurality of potential data postings or published postings to identify adverse event patterns, and wherein said analysis includes identifying a pattern of unstructured terms included in potential postings of a plurality of patients, and wherein said analysis (134) includes using natural language processing NLP techniques.

4. A method as claimed in any preceding claim, comprising the further step of initiating a global search of clinical trial databases for both additional indicators of the adverse events and for potential remedies.

5. A method as claimed in any preceding claim, comprising performing (131-134) semantic analysis of keywords based on publicly available dictionaries and thesauri and taxonomy lookups through similarities and stemming methods to initiate a globalised search.

6. A method as claimed in any preceding claim, wherein the network (1) includes a least one curator communication device (12) which performs tasks relating to the quality of the data in the network, and also relating to privacy associated with any publicly-generated and available data.

7. A method as claimed in any preceding claim, wherein said at least one digital data processor (10-15) performs real time privacy analysis (503) by activating additional processors as required and determined by the volume of postings being submitted, enabled by a micro services architecture utilising self-healing and annealing infrastructures, deploying new instances as detected by management software.

8. A method as claimed in any preceding claim, wherein the step (e) includes automatically obfuscating (507) values and terms to avoid excessive feedback to patient devices.

9. A method as claimed in claim 8, wherein said step (507) of obfuscating values and terms includes replacing a term with a random set of characters or with a pre-configured string which may have been specified as part of the study/patient setup.

10. A clinical trial network (1) comprising digital data processors (3, 10-15) user interfaces, a database server, and databases (11), wherein at least one said digital data processor is configured to perform steps of:

(a) performing patient data attribute classification during clinical trial setup (120) by identifying (121, 122) attributes which are sensitive, said attributes being instantiated as unstructured terms in clinical trial records, and applying a handle for use by a patient when engaging with the clinical trial network, said handle not having any of said sensitive attributes;
(b) providing a sensitivity risk weighting (132, 133) associated with each of a plurality of attributes, the risk weighting representing a risk value for the associated attribute and being in a range from a minimum to a maximum;
(c) receiving (501, 502) a potential data posting;
(d) performing real time privacy analysis (503) of the potential data posting, by identifying terms and posting parameters and using said risk weightings of attributes to determine a posting data risk level (Low, Medium, High) for the potential data posting,

a parameter is one or more selected from:

extracted metadata about the potential posting,
a set of automatically-extracted keywords,
a tag added or selected manually by a patient,
a flag to indicate the output of a preliminary automated privacy analysis, and
structured data resulting from natural language processing and automated semantic keyword extraction,

wherein the parameters in step (d) include derived parameters for the potential data posting to determine said posting risk level, in which a derived parameter is one or more selected from:

an unstructured term frequency in the posting,
a count of instances of each unstructured term in the posting, and
a total count of instances of unstructured terms which are potentially sensitive,

wherein the real time privacy analysis (503) is performed by a server executing an algorithm as follows:

$$risk(t,d) \approx \max_{0 \leq x \leq 1} \sum tfidf(t,d,D) = \max_{0 \leq x \leq 1} \sum_{t}^{n} tf(t,d) * idf(t,D)$$

in which the term frequency part of the algorithm is modified to take into account the privacy risk or importance associated with the specific attribute:

$$tf(t,d) = \sum_{i=1}^{n} tf_i * weight_i \Big/ T$$

where $tf(t,d)$ is a modified measure of the term frequency associated with a posting in a document d,
$tf_i$ is a count of instances of a term $i$ in the posting,
$weight_i$ is the risk or importance of the term $I$, and
$T$ is the total count off instances of the terms identified as being potentially sensitive occurring in the current document,
and in which $idf(t,D)$ denotes inverse document frequency, which is a measure of how unique a term is across all other postings published to date, and thus how much information is exposed by use of the attribute in a specific posting, and is given as:

$$idf(t,D) = \log \frac{N}{1 + |\{d \in D, t \in d\}|}$$

where $N$ is the total number of published postings
and $|\{d \in D, t \in d\}|$ is the number of postings where the term $d$ appears,

(e) posting data (505, 508, 509) according to the privacy analysis of step (d) as:

if said risk level is low, an unaltered version (505) of the original potential posting;
if the risk level is medium, an edited version (508) of the original potential posting;
if the risk level is high, reject the potential data posting; and

wherein the data processors automatically execute steps (d) and (e) for each potential data posting,
wherein the database server (11) persists the received potential data posting and associated data including inputs from contributors related to the received posting, and metadata, and also outputs for the received posting arising from step (d).

**Patentansprüche**

1. Verfahren, das von einem Klinische-Studien-Netzwerk (1) durchgeführt wird, das Digitaldatenprozessoren (10-15), Benutzeroberflächen, einen Datenbankserver und Datenbanken (11) umfasst, wobei das Verfahren (100) von mindestens einem genannten digitalen Datenprozessor durchgeführte Schritte beinhaltet, die Folgendes umfassen:

   (a) Durchführen einer Patientendaten-Attributklassifizierung beim Einrichten (120) einer klinischen Studie durch Identifizieren (121, 122) von Attributen, die sensibel sind, wobei die genannten Attribute als unstrukturierte Begriffe in Klinische-Studie-Datensätzen instanziiert werden, und Anwenden eines Handles zur Benutzung durch einen Patienten beim Tätigwerden in dem Klinische-Studien-Netzwerk, wobei das genannte Handle keines der genannten sensiblen Attribute aufweist;
   (b) Bereitstellen einer Sensitivitätsrisikogewichtung (132, 133) in Assoziation mit jedem von mehreren Attributen, wobei die Risikogewichtung einen Risikowert für das assoziierte Attribut darstellt und in einem Bereich von einem Minimum bis zu einem Maximum liegt;
   (c) Empfangen (501, 502) eines potentiellen Datenpostings;
   (d) Durchführen einer Echtzeit-Datenschutzanalyse (503) des potenziellen Datenpostings durch Identifizieren von Begriffen und Posting-Parametern und Verwenden der genannten Risikogewichtungen von Attributen, um eine Postingdaten-Risikostufe (Niedrig, Mittel, Hoch) für das potenzielle Datenposting zu bestimmen,

   ein Parameter ist einer oder mehrere ausgewählt aus:

   extrahierten Metadaten über das potenzielle Posting,
   einem Satz von automatisch extrahierten Schlüsselwörtern,
   einem von einem Patienten manuell hinzugefügten oder ausgewählten Tag,
   einem Flag zum Anzeigen des Ausgangs einer vorläufigen automatischen Datenschutzanalyse, und
   strukturierten Daten, die aus der Verarbeitung natürlicher Sprache und der automatischen Extraktion semantischer Schlüsselwörter resultieren,

   wobei die Parameter in Schritt (d) abgeleitete Parameter für das potenzielle Datenposting zum Bestimmen der genannten Posting-Risikostufe beinhalten, wobei ein abgeleiteter Parameter einer oder mehrere ist, die ausgewählt sind aus:

   einer Häufigkeit von unstrukturierten Begriffen in dem Posting,
   einer Anzahl von Instanzen jedes unstrukturierten Begriffs in dem Posting, und
   einer Gesamtzahl von Instanzen von unstrukturierten Begriffen, die potenziell sensibel sind,
   wobei die Echtzeit-Datenschutzanalyse (503) erfolgt, indem ein Server einen Algorithmus wie folgt ausführt:

$$risk(t,d) \approx \max_{0 \leq x \leq 1} \sum tfidf(t,d,D) = \max_{0 \leq x \leq 1} \sum_{t}^{n} tf(t,d) * idf(t,D)$$

   wobei der Begriffhäufigkeitsteil des Algorithmus geändert wird, um das/die mit dem spezifischen Attribut assoziierte Datenschutzrisiko oder Wichtigkeit zu berücksichtigen:

$$tf(t,d) = \sum_{i=1}^{n} tf_i * weight_i \Big/ T$$

   wobei $tf(t,d)$ ein modifiziertes Maß für die mit einem Posting in einem Dokument $d$ assoziierte Begriffshäufigkeit ist,
   $tf_i$ die Anzahl der Instanzen eines Begriffs $i$ in dem Posting ist,
   $weight_i$ [Gewichtung $_i$] das Risiko oder die Wichtigkeit des Begriffs $I$ ist, und
   $T$ die Gesamtzahl der Instanzen der als potenziell sensibel identifizierten Begriffe ist, die im aktuellen Dokument vorkommen,
   und wobei $idf(t,D)$ die inverse Dokumentenhäufigkeit bezeichnet, die ein Maß dafür ist, wie einzigartig ein Begriff über alle anderen bisher veröffentlichten Postings ist, und somit dafür, wie viele Informationen durch die Verwendung des Attributs in einem bestimmten Posting preisgegeben werden, und die wie folgt angegeben wird:

$$idf(t,D) = \log \frac{N}{1 + |\{d \in D, t \in d\}|}$$

wobei $N$ die Gesamtzahl der veröffentlichten Postings ist und $|\{d \in D, t \in d\}|$ die Anzahl von Postings ist, in denen der Begriff $d$ vorkommt,

(e) Posten von Daten (505, 508, 509) gemäß der Datenschutzanalyse von Schritt (d) als:

wenn die genannte Risikostufe niedrig ist, eine unveränderte Version (505) des ursprünglichen potenziellen Postings;
wenn die Risikostufe mittel ist, eine bearbeitete Version (508) des ursprünglichen potenziellen Postings;
wenn die Risikostufe hoch ist, Ablehnen des potentiellen Datenpostings; und
wobei der mindestens eine Digitaldatenprozessor automatisch die Schritte (d) und (e) für jedes potentielle Datenposting ausführt, und
wobei der Datenbankserver (11) das empfangene potenzielle Datenposting und assoziierte Daten, einschließlich Eingaben von Mitwirkenden in Bezug auf das empfangene Posting und Metadaten sowie sich aus Schritt (d) ergebende Ausgaben für das empfangene Posting, aufbewahrt.

2. Verfahren nach Anspruch 1, wobei Schritt (a) und/oder (b) als Reaktion auf die prozessorgeführte Eingabe von Daten durch einen Patienten gemäß einer Patientendatenschnittstelle durchgeführt wird/werden, und wobei Schritt (a) pro klinischer Studie als Teil einer Klinische-Studie-Planungsphase (120) durchgeführt wird.

3. Verfahren nach Anspruch 1 oder 2, wobei der genannte mindestens eine Digitaldatenprozessor zum Analysieren mehrerer potenzieller Datenpostings oder veröffentlichter Postings konfiguriert ist, um unerwünschte Ereignismuster zu identifizieren, und wobei die genannte Analyse das Identifizieren eines Musters von unstrukturierten Begriffen umfasst, die in potenziellen Postings mehrerer Patienten enthalten sind, und wobei die genannte Analyse (134) die Nutzung von NLP-(Natural Language Processing)-Techniken beinhaltet.

4. Verfahren nach einem vorherigen Anspruch, das den weiteren Schritt des Einleitens einer globalen Suche sowohl nach zusätzlichen Indikatoren für die unerwünschten Ereignisse als auch nach möglichen Abhilfemaßnahmen in Klinische- Studien-Datenbanken beinhaltet.

5. Verfahren nach einem vorherigen Anspruch, das das Durchführen (131-134) einer semantischen Analyse von Schlüsselwörtern auf der Basis von öffentlich zugänglichen Wörterbüchern und Thesauri und Taxonomie-Suchvorgängen durch Ähnlichkeits- und Stemming-Methoden beinhaltet, um eine globalisierte Suche einzuleiten.

6. Verfahren nach einem vorherigen Anspruch, wobei das Netzwerk (1) mindestens ein Kurator-Kommunikationsgerät (12) enthält, das Aufgaben in Bezug auf die Qualität der Daten im Netzwerk und auch in Bezug auf Datenschutz in Assoziation mit öffentlich erzeugten und verfügbaren Daten durchführt.

7. Verfahren nach einem vorherigen Anspruch, wobei der genannte mindestens eine Digitaldatenprozessor (10-15) Echtzeit-Datenschutzanalyse (503) durch Aktivieren zusätzlicher Prozessoren nach Bedarf und bestimmt durch das Volumen von submittierten Postings, ermöglicht durch eine Mikroservice-Architektur, die selbstheilende und anellierende Infrastrukturen nutzt, unter Einsatz neuer Instanzen wie von einer Verwaltungssoftware erkannt, durchführt.

8. Verfahren nach einem vorherigen Anspruch, wobei Schritt (e) das automatische Obfuskieren (507) von Werten und Begriffen beinhaltet, um übermäßiges Feedback an Patientengeräte zu vermeiden.

9. Verfahren nach Anspruch 8, wobei der genannte Schritt (507) des Obfuskierens von Werten und Begriffen das Ersetzen eines Begriffs durch einen zufälligen Satz von Zeichen oder durch eine vorkonfigurierte Zeichenkette beinhaltet, die möglicherweise als Teil des Studien/Patienten-Setups angegeben wurde.

10. Klinische-Studien-Netzwerk (1), das Digitaldatenprozessoren (3, 10-15), Benutzeroberflächen, einen Datenbankserver und Datenbanken (11) umfasst, wobei mindestens ein genannter Digitaldatenprozessor so konfiguriert ist, dass er die folgenden Schritte durchführt:

(a) Durchführen einer Patientendaten-Attributklassifizierung beim Einrichten (120) einer klinischen Studie durch Identifizieren (121, 122) von Attributen, die sensibel sind, wobei die genannten Attribute als unstrukturierte Begriffe in Klinische-Studie-Datensätzen instanziiert werden, und Anwenden eines Handles zur Benutzung durch einen Patienten beim Tätigwerden in dem Klinische-Studien-Netzwerk, wobei das genannte Handle keines der genannten sensiblen Attribute aufweist;

(b) Bereitstellen einer Sensitivitätsrisikogewichtung (132, 133) in Assoziation mit jedem von mehreren Attributen, wobei die Risikogewichtung einen Risikowert für das assoziierte Attribut darstellt und in einem Bereich von einem Minimum bis zu einem Maximum liegt;

(c) Empfangen (501, 502) eines potenziellen Datenpostings;

(d) Durchführen einer Echtzeit-Datenschutzanalyse (503) des potenziellen Datenpostings durch Identifizieren von Begriffen und Posting-Parametern und Verwenden der genannten Risikogewichtungen von Attributen zum Bestimmen einer Postingdaten-Risikostufe (Niedrig, Mittel, Hoch) für das potenzielle Datenposting,

ein Parameter ist einer oder mehrere ausgewählt aus:

extrahierten Metadaten über das potenzielle Posting,
einem Satz von automatisch extrahierten Schlüsselwörtern,
einem von einem Patienten manuell hinzugefügten oder ausgewählten Tag,
einem Flag zum Anzeigen des Ausgangs einer vorläufigen automatischen Datenschutzanalyse, und
strukturierten Daten, die aus der Verarbeitung natürlicher Sprache und der automatischen Extraktion semantischer Schlüsselwörter resultieren,

wobei die Parameter in Schritt (d) abgeleitete Parameter für das potenzielle Datenposting zum Bestimmen der genannten Posting-Risikostufe beinhalten, wobei ein abgeleiteter Parameter einer oder mehrere ist, die ausgewählt sind aus:

einer Häufigkeit von unstrukturierten Begriffen in dem Posting,
einer Anzahl von Instanzen jedes unstrukturierten Begriffs in dem Posting, und
einer Gesamtzahl von Instanzen von unstrukturierten Begriffen, die potenziell sensibel sind,
wobei die Echtzeit-Datenschutzanalyse (503) von einem Server durchgeführt wird, der einen Algorithmus wie folgt ausführt:

$$risk(t,d) \approx \max_{0 \leq x \leq 1} \sum tfidf(t,d,D) = \max_{0 \leq x \leq 1} \sum_{t}^{n} tf(t,d) * idf(t,D)$$

wobei der Begriffhäufigkeitsteil des Algorithmus geändert wird, um das/die mit dem spezifischen Attribut assoziierte Datenschutzrisiko oder Wichtigkeit zu berücksichtigen:

$$tf(t,d) = \sum_{i=1}^{n} tf_i * weight_i / T$$

wobei $tf(t,d)$ ein modifiziertes Maß für die mit einem Posting in einem Dokument $d$ assoziierte Begriffshäufigkeit ist,
$tf_i$ die Anzahl der Instanzen eines Begriffs $i$ in dem Posting ist,
$weight_i$ [Gewichtung $_i$] das Risiko oder die Wichtigkeit des Begriffs $I$ ist, und
$T$ die Gesamtzahl der Instanzen der als potenziell sensibel identifizierten Begriffe ist, die im aktuellen Dokument vorkommen,
und wobei $idf(t,D)$ die inverse Dokumentenhäufigkeit bezeichnet, die ein Maß dafür ist, wie einzigartig ein Begriff über alle anderen bisher veröffentlichten Postings ist, und somit dafür, wie viele Informationen durch die Verwendung des Attributs in einem bestimmten Posting preisgegeben werden, und die wie folgt angegeben wird:

$$idf(t, D) = \log \frac{N}{1 + |\{d \in D, t \in d\}|}$$

wobei $N$ die Gesamtzahl der veröffentlichten Postings ist
und $|\{d \in D, t \in d\}|$ die Anzahl von Postings ist, in denen der Begriff $d$ vorkommt,

(e) Posten von Daten (505, 508, 509) gemäß der Datenschutzanalyse von Schritt (d) als:

wenn die genannte Risikostufe niedrig ist, eine unveränderte Version (505) des ursprünglichen potenziellen Postings;
wenn die Risikostufe mittel ist, eine bearbeitete Version (508) des ursprünglichen potenziellen Postings;
wenn die Risikostufe hoch ist, Ablehnen des potentiellen Datenpostings; und
wobei die Digitaldatenprozessoren automatisch die Schritte (d) und (e) für jedes potentielle Datenposting ausführen,
wobei der Datenbankserver (11) das empfangene potenzielle Datenposting und assoziierte Daten, einschließlich Eingaben von Mitwirkenden in Bezug auf das empfangene Posting und Metadaten sowie sich aus Schritt (d) ergebende Ausgaben für das empfangene Posting, aufbewahrt.

**Revendications**

1. Un procédé exécuté par un réseau d'essais cliniques (1) comprenant des interfaces utilisateurs de processeurs de données numériques (10-15), un serveur de bases de données et des bases de données (11), où le procédé (100) comprend des opérations exécutées par au moins un dit processeur de données numériques comprenant :

(a) l'exécution d'un classement d'attributs de données de patient au cours d'un établissement d'essai clinique (120) par l'identification (121, 122) d'attributs qui sont sensibles, lesdits attributs étant instanciés sous la forme de termes non structurés dans des enregistrements d'essais cliniques, et l'application d'un pseudonyme pour une utilisation par un patient lorsqu'il entre en relation avec le réseau d'essais cliniques, ledit pseudonyme ne possédant aucun desdits attributs sensibles,
(b) la fourniture d'une pondération de risque de sensibilité (132, 133) associée à chaque attribut d'une pluralité d'attributs, la pondération de risque représentant une valeur de risque pour l'attribut associé et étant dans une plage d'un minimum à un maximum,
(c) la réception (501, 502) d'une publication de données potentielle,
(d) l'exécution d'une analyse de confidentialité en temps réel (503) de la publication de données potentielle par l'identification de termes et de paramètres de publication et l'utilisation desdites pondérations de risque d'attributs de façon à déterminer un niveau de risque de données de publication (faible, moyen, élevé) pour la publication de données potentielle,

un paramètre est un ou plusieurs éléments sélectionnés parmi :

des métadonnées extraites relatives à la publication potentielle,
un ensemble de mots-clés extraits automatiquement,
une étiquette ajoutée ou sélectionnée manuellement par un patient,
une balise destinée à indiquer la production en sortie d'une analyse de confidentialité automatisée préliminaire, et
des données structurées résultant d'un traitement de langage naturel et d'une extraction de mots-clés sémantiques automatisée,

où les paramètres à l'opération (d) comprennent des paramètres dérivés destinés à la publication de données potentielle de façon à déterminer ledit niveau de risque de publication, où un paramètre dérivé est un ou plusieurs éléments sélectionnés parmi,

une fréquence de termes non structurés dans la publication,
un décompte d'instances de chaque terme non structuré dans la publication, et
un décompte total d'instances de termes non structurés qui sont potentiellement sensibles,

où l'analyse de confidentialité en temps réel (503) est exécutée par un serveur exécutant un algorithme comme suit :

$$risk(t,d) \approx \max_{0 \leq x \leq 1} \sum tfidf(t,d,D) = \max_{0 \leq x \leq 1} \sum_{t}^{u} tf(t,d) * idf(t,D)$$

dans lequel la partie fréquence de terme de l'algorithme est modifiée de façon à prendre en compte l'importance ou le risque de confidentialité associé à l'attribut spécifique :

$$tf(t,d) = \sum_{i=1}^{n} tf_i * weight_i \Big/ T$$

où $tf(t,d)$ est une mesure modifiée de la fréquence de terme associée à une publication dans un document d, $tf_i$ est un décompte d'instances d'un terme $i$ dans la publication, $weight_i$ [$poids_i$] est le risque ou l'importance du terme $I$, et

$T$ est le décompte total d'instances des termes identifiés être potentiellement sensibles rencontrés dans le document courant,

et dans lequel $idf(t,D)$ désigne une fréquence de document inverse, qui est une mesure du caractère unique d'un terme sur la totalité des autres publications publiées à ce jour, et en conséquence la quantité d'informations qui est exposée par l'utilisation de l'attribut dans une publication spécifique, et est donnée sous forme :

$$idf(t,D) = \log \frac{N}{1 + |\{d \in D, t \in d\}|}$$

où $N$ est le nombre total de publications publiées
et $|\{d \in D, t \in d\}|$ est le nombre de publications dans lesquelles le terme d apparaît,
(e) la publication de données (505, 508, 509) en fonction de l'analyse de confidentialité de l'opération (d) sous la forme de :

si ledit niveau de risque est faible, une version non altérée (505) de la publication potentielle d'origine,
si le niveau de risque est moyen, une version éditée (508) de la publication potentielle d'origine,
si le niveau de risque est élevée, rejeter la publication de données potentielle, et

où le au moins un processeur de données numériques exécute automatiquement les opérations (d) et (e) pour chaque publication de données potentielle, et où le serveur de bases de données (11) conserve la publication de données potentielle reçue et des données associées comprenant des entrées provenant de contributeurs reliés à la publication reçue, et des métadonnées, et également des productions en sortie pour la publication reçue provenant de l'opération (d).

**2.** Un procédé selon la Revendication 1, où une opération ou les deux parmi les opérations (a) et (b) sont exécutées en réponse à une entrée de données, guidée par un processeur, par un patient en fonction d'une interface de données de patient, et où l'opération (a) est exécutée par essai clinique dans le cadre d'une phase de conception d'essai clinique (120).

**3.** Un procédé selon les Revendications 1 ou 2, où ledit au moins un processeur de données numériques est configuré de façon à analyser une pluralité de publications de données potentielles ou de publications publiées de façon à identifier des modèles d'événements adverses, et où ladite analyse comprend l'identification d'un modèle de termes non structurés inclus dans des publications potentielles d'une pluralité de patients, et où ladite analyse (134) comprend l'utilisation de techniques de traitement de langage naturel, NLP.

**4.** Un procédé selon l'une quelconque des Revendications précédentes, comprenant l'opération complémentaire de lancement d'une recherche globale dans des bases de données d'essais cliniques à la fois d'indicateurs additionnels des événements adverses et de remèdes potentiels.

5. Un procédé selon l'une quelconque des Revendications précédentes, comprenant l'exécution (131-134) d'une analyse sémantique de mots-clés en fonction de consultations dans des thésaurus, des taxonomies et des dictionnaires publiquement disponibles par l'intermédiaire de procédés de recherche de radicaux et de similarités de façon à lancer une recherche globalisée.

6. Un procédé selon l'une quelconque des Revendications précédentes, où le réseau (1) comprend au moins un dispositif de communication de conservateur (12) qui exécute des tâches relatives à la qualité des données dans le réseau, et également relatives à une confidentialité associée à toutes données générées et disponibles publiquement.

7. Un procédé selon l'une quelconque des Revendications précédentes, où ledit au moins un processeur de données numériques (10-15) exécute une analyse de confidentialité en temps réel (503) par l'activation de processeurs additionnels tels que requis et déterminés par le volume de publications qui ont été soumises, permise par une architecture de micro-services utilisant des infrastructures d'auto-rétablissement et de recuisson, déployant de nouvelles instances telles que détectées par un logiciel de gestion.

8. Un procédé selon l'une quelconque des Revendications précédentes, où l'opération (e) comprend la dissimulation automatique (507) de valeurs et de termes de façon à éviter des informations en retour excessives vers des dispositifs de patient.

9. Un procédé selon la Revendication 8, où ladite opération (507) de dissimulation de valeurs et de termes comprend le remplacement d'un terme par un ensemble aléatoire de caractères ou par une chaîne préconfigurée qui peut avoir été spécifiée dans le cadre de l'établissement d'étude/patient.

10. Un réseau d'essais cliniques (1) comprenant des interfaces utilisateurs de processeurs de données numériques (3, 10-15), un serveur de bases de données et des bases de données (11), où au moins un dit processeur de données numériques est configuré de façon à exécuter les opérations suivantes :

(a) l'exécution d'un classement d'attributs de données de patient au cours d'un établissement d'essai clinique (120) par l'identification (121, 122) d'attributs qui sont sensibles, lesdits attributs étant instanciés sous la forme de termes non structurés dans des enregistrements d'essais cliniques, et l'application d'un pseudonyme pour une utilisation par un patient lorsqu'il entre en relation avec le réseau d'essais cliniques, ledit pseudonyme ne possédant aucun desdits attributs sensibles,
(b) la fourniture d'une pondération de risque de sensibilité (132, 133) associée à chaque attribut d'une pluralité d'attributs, la pondération de risque représentant une valeur de risque pour l'attribut associé et étant dans une plage d'un minimum à un maximum,
(c) la réception (501, 502) d'une publication de données potentielle,
(d) l'exécution d'une analyse de confidentialité en temps réel (503) de la publication de données potentielle, par l'identification de termes et de paramètres de publication et l'utilisation desdites pondérations de risque d'attributs de façon à déterminer un niveau de risque de données de publication (faible, moyen, élevé) pour la publication de données potentielle,

un paramètre est un ou plusieurs éléments sélectionnés parmi :

des métadonnées extraites relatives à la publication potentielle,
un ensemble de mots-clés extraits automatiquement,
une étiquette ajoutée ou sélectionnée manuellement par un patient,
une balise destinée à indiquer la production en sortie d'une analyse de confidentialité automatisée préliminaire, et
des données structurées résultant d'un traitement de langage naturel et d'une extraction de mots-clés sémantiques automatisée,

où les paramètres à l'opération (d) comprennent des paramètres dérivés pour la publication de données potentielle de façon à déterminer ledit niveau de risque de publication, où un paramètre dérivé est un ou plusieurs éléments sélectionnés parmi :

une fréquence de termes non structurés dans la publication,
un décompte d'instances de chaque terme non structuré dans la publication, et
un décompte total d'instances de termes non structurés qui sont potentiellement sensibles,

où l'analyse de confidentialité en temps réel (503) est exécutée par un serveur exécutant un algorithme comme suit :

$$risk(t,d) \approx \max_{0 \leq x \leq 1} \sum tfidf(t,d,D) = \max_{0 \leq x \leq 1} \sum_{t}^{n} tf(t,d) * idf(t,D)$$

dans lequel la partie fréquence de terme de l'algorithme est modifiée de façon à prendre en compte l'importance ou le risque de confidentialité associé à l'attribut spécifique :

$$tf(t,d) = \sum_{i=1}^{n} tf_i * weight_i / T$$

où *tf(t,d)* est une mesure modifiée de la fréquence de terme associée à une publication dans un document d, *tf_i* est un décompte d'instances d'un terme *i* dans la publication, *weight_i [poids_i]* est le risque ou l'importance du terme *I*, et
*T* est le décompte total d'instances des termes identifiés être potentiellement sensibles rencontrés dans le document courant,
et dans lequel *idf(t,D)* désigne une fréquence de document inverse, qui est une mesure du caractère unique d'un terme sur la totalité des autres publications publiées à ce jour, et en conséquence la quantité d'informations qui est exposée par l'utilisation de l'attribut dans une publication spécifique, et est donnée sous forme :

$$idf(t,D) = \log \frac{N}{1 + |\{d \in D, t \in d\}|}$$

où *N* est le nombre total de publications publiées,
et |{*d* ∈ *D, t* ∈ *d*}| est le nombre de publications dans lesquelles le terme d apparaît,

(e) la publication de données (505, 508, 509) en fonction de l'analyse de confidentialité de l'opération (d) sous la forme de :

si ledit niveau de risque est faible, une version non altérée (505) de la publication potentielle d'origine,
si le niveau de risque est moyen, une version éditée (508) de la publication potentielle d'origine,
si le niveau de risque est élevé, rejeter la publication de données potentielle, et

où les processeurs de données exécutent automatiquement les opérations (d) et (e) pour chaque publication de données potentielle, où le serveur de bases de données (11) conserve la publication de données potentielle reçue et des données associées comprenant des entrées provenant de contributeurs reliés à la publication reçue, et des métadonnées, et également des productions en sortie pour la publication reçue provenant de l'opération (d).

Fig.1

Fig.2

Fig.3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20050065824 A
- US 20040199781 A
- US 8715180 B
- US 8606746 B
- US 8650645 B
- US 20060059149 A
- US 20010027331 A
- US 20150161397 A

**Non-patent literature cited in the description**

- **SALTON, GERARD ; CHRISTOPHER BUCKLEY.** *Term-weighting approaches in automatic text retrieval,* 1988, 5
- **AIZAWA, AKIKO.** *An information-theoretic perspective of tf-idf measures,* 2003, 1
- International Telegraph and Telephone Consultative Committee (CCITT). Information Technology - Open Systems Interconnection - Systems Management: Alarm Reporting Function. *International Telecommunication Union,* 10 February 1992, https://www.itu.int/rec/T-REC-X.733/en